# EUROPÄISCHE PATENTSCHRIFT

(11) **EP 2 648 799 B1**
(45) Veröffentlichungstag und Bekanntmachung des Hinweises auf die Patenterteilung: **19.02.2014**
(21) Anmeldenummer: 11788069.0
(22) Anmeldetag: 25.11.2011
(51) Int. Cl.: A61N 1/36, A61N 1/04

(54) **ELEKTRODENANORDNUNG**
ELECTRODE ARRANGEMENT
AGENCEMENT D'ÉLECTRODES

(30) Priorität: 12.12.2010 DE 102010054165
(43) Veröffentlichungstag der Anmeldung: 16.10.2013
(73) Patentinhaber: Cerbomed GmbH, 91052 Erlangen (DE)
(72) Erfinder: BECK, Christoph, 91096 Möhrendorf (DE); BÄR, Stefan, 90556 Cadolzburg (DE)
(74) Vertreter: Gosdin, Michael
(86) Internationale Anmeldenummer: PCT/EP2011/005934
(87) Internationale Veröffentlichungsnummer: WO 2012/079700

(56) Entgegenhaltungen:
- WO-A1-2007/134804
- WO-A2-2008/042863
- WO-A2-2009/155516

## Beschreibung

Die Erfindung betrifft eine Elektrodenanordnung zur Aufbringung eines transkutanen elektrischen Stimulationsreizes auf die Oberfläche eines Abschnittes des menschlichen Ohrs, die ein am oder im Ohr anbringbares Halteelement sowie mindestens eine Elektrode aufweist, die in einem Elektrodenträger angeordnet ist.

Es ist generell bekannt, durch invasive und non-invasive Reizung der Nerven Einfluss auf deren neurophysiologische und neuroelektrische Qualität und damit auf die Funktion der stimulierten Nerven zu nehmen. Hierdurch können verschiedene Krankheitszustände behandelt werden. Es existieren zahlreiche Vorrichtungen sowohl zu invasiven als auch zu non-invasiven Stimulation.

Die vorliegende Erfindung stellt auf die Methode der transkutanen elektrischen Nervenstimulation ab. Bei diesem Verfahren werden Impulsströme verschiedener Stromformen, Amplituden, Impulsdauern und Frequenzen durch die Haut hindurch an verschiedenen Nerven appliziert und verändern deren Statusparameter in vorteilhafter Weise.

Eine Elektrodenanordnung der eingangs genannten Art ist aus der EP 2 026 872 B1 bekannt. Hier ist eine Elektrodenanordnung beschrieben, die ein vollständig in der Pinna (Ohrmuschel) unterbringbares Gehäuse umfasst. Von diesem aus erstrecken sich zwei gebogene, drahtförmige Abschnitte, wobei diese als federelastische Halterungen ausgebildet sind. Damit kann die Elektrodenanordnung durch sanftes Einklemmen in der Pinna in die benötigte Position gebracht werden, so dass der Gehörgang mit einem transkutanen Stimulationsreiz beaufschlagt werden kann. Andere Stimulationsvorrichtungen offenbaren die DE 10 2005 003 735 A1 und die US 5 514 175**.**

Eine andere Stimulationsvorrichtung offenbart die WO 2009/155516 A2**,** wobei die Elektrodenhalterung nach Art eines Kopfhörers gestaltet ist. Die WO 2008/042863 A2 beschreibt eine Stimulationsvorrichtung, bei der eine implantierte Elektrode zum Einsatz kommt.

Wenngleich die oben genannte vorbekannte Elektrodenanordnung bereits zu guten Behandlungsergebnissen führt, haben sich in der Praxis gewisse Nachteile der Anordnung herausgestellt.

Da naturbedingt die Elektrodenanordnungen mit ihren Elektroden sehr klein bauen müssen, ist die mechanische Stabilität der benötigten Verbindungen, insbesondere der Elektroden mit ihren Anschlusskabeln, bisher nicht optimal gelöst. Daher kann es zu einer ungenügenden Halterung der Elektroden kommen, was dadurch begünstigt wird, dass ein sehr weiches anschmiegsames Material (insbesondere Silikon) für die Halterung eingesetzt werden muss, um einen hinreichenden Tragekomfort zu erzielen. Mitunter kommt es bei entsprechender mechanischer Belastung der Elektrodenanordnung bei ihrem Einsetzen in den Gehörgang zu irreparablen Schäden an den Kontakten der Elektroden.

Des weiteren wurde es als nachteilig erkannt, dass es schwer ist, eine bestehende Elektrodenanordnung gegebenenfalls auf die individuelle Größe der Pinna bzw. generell des Ohrs anzupassen. Dies führt mitunter zu hohem Aufwand beim Anlegen der Elektrodenanordnung bzw. zu einem nicht optimalen Tragekomfort.

Nachteilig ist es weiterhin bei manchen vorbekannten Elektrodenanordnungen, dass das Hörvermögen negativ beeinflusst werden kann.

Der Erfindung liegt daher die **Aufgabe** zugrunde, eine Elektrodenanordnung der gattungsgemäßen Art so fortzubilden, dass die genannten Nachteile überwunden werden. Es soll also eine Elektrodenanordnung vorgeschlagen werden, mit der auch größere mechanische Belastungen sicher übertragen werden können, wobei die Elektroden sicher in Position gehalten werden können. Dabei soll vor allem eine einfache Möglichkeit geschaffen werden, die Elektrodenanordnung an unterschiedliche Größen des Ohres anpassen zu können. Das Anlegen der Elektrodenanordnung soll in einfacher und bequemer Weise möglich sein. Das Hörvermögen soll dabei beim Tagen der Elektrodenanordnung so wenig wie möglich negativ beeinflusst werden.

Die **Lösung** dieser Aufgabe wird durch eine Vorrichtung gemäß Anspruch 1 erreicht.

Der Elektrodenträger ist zumeist an einem axialen Ende der Haltestange angeordnet.

Der Elektrodenträger weist, insbesondere als Elektrodenkopf ausgebildet, bevorzugt mindestens eine Stimulationselektrode und mindestens eine Referenzelektrode auf.

Die Linearführung des Halteelements kann durch eine Ausnehmung gebildet werden, die in einem Schnitt senkrecht zur Längsachse entlang der Längsachse eine gleichbleibende Form, insbesondere eine Kreisform, aufweist; die Haltestange weist dann bevorzugt zumindest über einen Teil ihrer Erstreckung einen kreisförmige Querschnitt auf.

Weiterhin können Rastmittel zwischen der Linearführung und der Haltestange wirksam angeordnet sein, so dass die Haltestange relativ zur Linearführung entlang der Längsachse in vorgegebenen Relativpositionen verrastbar angeordnet werden kann. Die Rastmittel können mindestens eine nutförmige Ausnehmung in der Linearführung umfassen sowie mindestens einen sich radial nach außen erstreckenden Vorsprung in der Haltestange. Die Vorsprünge sind vorzugsweise als Wellenbahn mit einer Anzahl radialer Erhebungen ausgebildet.

Ferner können additiv oder alternativ auch Feststellmittel vorgesehen werden, die die relative lineare Position zwischen dem Halteelement und der Haltestange blockieren, d. h. die Linearverschiebung mittels der Linearführung verhindern.

Weiterhin können Federmittel zwischen der Linearführung und der Haltestange wirksam angeordnet sein, so dass die Haltestange relativ zur Linearführung in Richtung der Längsachse elastisch vorspannbar ist.

Fernem können Mittel zur Verhinderung einer Verdrehung der Haltestange relativ zur Linearführung um die Längsachse vorhanden sein. Diese Mittel können durch einen sich radial von der Haltestange weg erstreckenden Seitenfortsatz gebildet werden, wobei in der Linearführung eine korrespondierende Ausnehmung für den Seitenfortsatz ausgebildet ist. Der Seitenfortsatz sowie die Ausnehmung können in einem Schnitt senkrecht zur Längsachse eine rechteckige Form aufweisen. Am von der Haltestange entfernten Ende des Seitenfortsatzes können Haltemittel für ein elektrisches Kabel angeordnet sein, das mit der mindestens einen Elektrode in elektrischer Verbindung steht. Die Haltemittel sind bevorzugt als mindestens ein Haltering für das Kabel ausgebildet.

Die Haltestange samt dem Seitenfortsatz und gegebenenfalls den Haltemitteln sind bevorzugt als einstückiges Spritzgießformteil ausgebildet.

Die Haltestange hat in ihrem axialen Endbereich, in dem der Elektrodenträger angeordnet ist, bevorzugt eine S-förmige Ausbildung. Sie kann in ihrem axialen Endbereich, in dem der Elektrodenträger angeordnet ist, mit einem Federelement, insbesondere mit einem Federdraht oder einer Blattfeder, verstärkt sein, um eine hinreichende Elastizität des Elektrodenträgers relativ zum Halteelement sicherzustellen.

Generell kann vorgesehen werden, dass durch entsprechende Maßnahmen eine gewünschte Elastizität des Elektrodenträgers relativ zum Halteelement erreicht wird. Die genannte Integration eines Federelements ist hier nur eine Möglichkeit. Es kann durch geeignete konstruktive Maßnahmen gleichermaßen erreicht werden, dass die Elastizität bzw. Federkonstante in einem gewünschten Bereich liegt. So ist es beispielsweise möglich, einen Abschnitt der Haltestange durch eine metallische Blattfeder zu verstärken und gleichzeitig das Material der Haltestange in diesem Bereich zurückzunehmen oder ganz hierauf zu verzichten. Die Blattfeder ermöglicht eine Federelastizität in eine definierte Richtung, während die Verbindung zwischen dem Halteelement und dem Elektrodenträger in anderen Belastungsrichtungen steifer bleibt.

Aus dem oben Gesagten wird verständlich, dass der Begriff der "Haltestange" hier weit zu interpretieren ist und sich keinesfalls auf klassische stangenförmige Strukturen beschränkt.

Die Haltestange kann in ihrem axialen Endbereich, in dem der Elektrodenträger angeordnet ist, weiterhin mindestens eine seitliche Einkerbung oder mindestens einen seitlichen Einschnitt aufweisen, um im axialen Endbereich die Biegesteifigkeit der Haltestange um eine zur Längsachse senkrechte Richtung zu reduzieren. Die Anpassung des Elektrodenträgers an die Oberflächentopographie der zu stimulierenden Haut wird dadurch erleichtert.

Das Auflageteil hat bevorzugt einen ringförmigen Abschnitt, insbesondere einen kreisringförmigen Abschnitt oder einen ovalen Ringabschnitt.

Der ringförmige Abschnitt des Auflageteils kann dabei an mindestens einer Umfangsstelle eine Unterbrechung aufweisen. Damit kann erreicht werden, dass eine vereinfachte Anpassung des kreisringförmigen Abschnitts an den Auflagebereich im Ohr bei verschiedenen Ohrgrößen gegeben ist.

Eine alternative oder additive Möglichkeit, die Größe und/oder Form des ringförmigen Abschnitts an individuelle Bedürfnisse anzupassen, besteht darin, dass über den Außenumfang des ringförmigen Abschnitts ein ringartiger Überzugskörper (z. B. Gummiring) übergezogen wird. Dieser macht den Außenumfang des ringförmigen Abschnitts größer. Es ist aber auch möglich, dass der Überzugskörper eine asymmetrische Form hat, so dass hiermit auch die Außenform des ringförmigen Abschnitts verändert werden kann, um eine bessere Passgenauigkeit der Elektrodenanordnung im Ohr zu erreichen, d. h. die Anpassbarkeit der Elektrodenanordnung wird verbessert ermöglicht.

Zumindest der ringförmige Abschnitt des Auflageteils, gegebenenfalls auch der genannte Überzugskörper, kann aus einem elastischen, weichen Material bestehen, insbesondere aus einem biokompatiblen Elastomermaterial, vorzugsweise aus Silikon oder aus einem Material, das Silikon aufweist.

Das Halteelement kann ferner eine Wangenauflage umfassen, die an dem vom Elektrodenträger entfernten Ende der Elektrodenanordnung am Halteelement angeordnet ist. Damit kann eine verbesserte Abstützung der Elektrodenanordnung erreicht werden.

Die mindestens eine Elektrode ist bevorzugt in das Material des Elektrodenträgers zumindest teilweise eingebettet, insbesondere durch einen Spritzgießvorgang umspritzt.

Die mindestens eine Elektrode weist bevorzugt einen Kontaktabschnitt und einen stiftförmigen Verankerungsabschnitt auf, wobei zwischen dem Kontaktabschnitt und dem Verankerungsabschnitt mindestens ein Einschnitt angeordnet ist. Der stiftförmige Verankerungsabschnitt kann dabei an mindestens einer Umfangsstelle eine Abflachung oder eine Ausnehmung oder einen Vorsprung aufweisen, um eine Drehfestlegung beim Einbetten in das Material des Elektrodenträgers zu erzeugen.

Die Teile der Elektrodenanordnung bestehen bevorzugt - soweit Hautkontakt gegeben ist - aus einem weichen Material, wobei speziell an ein Elastomermaterial gedacht ist, insbesondere an Silikon oder an ein Material, das Silikon aufweist. Indes bestehen der Elektrodenkopf und die Haltestange bevorzugt aus einem thermoplastischen oder duroplastischen Kunststoffmaterial, beispielsweise auch aus Polyurethan.

Die Stimulationselektrode(n) und die Referenzelektrode(n) können in das Material des Elektrodenkopfs zumindest teilweise eingebettet sein. Sie können beim Spritzgießen des Elektrodenkopfs mit in das Spritzgießwerkzeug eingelegt und dann vom Material des Elektrodenkopfs umspritzt werden.

Es sei erwähnt, dass die vorgeschlagene Elektrodenanordnung auch nur eine Elektrode aufweisen kann. Möglich ist, es eine separate Referenzelektrode eingesetzt wird, die außerhalb der Elektrodenanordnung (z. B. hinter dem Ohr) platziert wird und mit dem Stimulationsgerät in elektrischer Verbindung steht. Möglich ist es auch, dass zwar - wie im Ausführungsbeispiel - zwei oder mehr Elektroden vorhanden sind, dass allerdings eine weitere Referenzelektrode außerhalb der Elektrodenanordnung zum Einsatz kommt.

Eine weitere Fortbildung sieht vor, dass die Elektrodenanordnung, vorzugsweise im Bereich des Auflageteils bzw. dessen ringförmigen Abschnitts mit einem akustischen Geber (Lautsprecher) versehen ist. Damit wird es möglich, auch akustische Signale während der Elektrostimulation dem Benutzer der Elektrodenanordnung zuzuleiten, was auch zu Unterhaltungszwecken geschehen kann.

Vorteilhaft ist es, dass durch die vorgeschlagene Ausgestaltung der Elektrodenanordnung eine recht stabile Konstruktion erreicht wird, ohne den Tragekomfort negativ zu beeinflussen. Die Elektroden werden stabil und zuverlässig in der benötigten Position gehalten, da sie über die Haltestange am relativ stabilen Halteelement angeordnet sind.

Durch die zweiteilige Bauweise ist es im Übrigen möglich, die Haltestange samt Elektrodenkopf auf der einen Seite mit dem Halteelement auf der anderen Seite zu kombinieren. Dies eröffnet die Möglichkeit, unterschiedlich große Elemente miteinander zu kombinieren. Eine bevorzugte Lieferausstattung sieht daher mehr als eine Haltestange samt Elektrodenkopf und/oder mehr als ein Halteelement auf. Der Anwender kann dann die Elemente mit der für seine Verhältnisse optimalen Größe auswählen und zusammenfügen, um eine optimal passende Elektrodenanordnung zur Verfügung zu haben.

Insbesondere die Halteelemente können so in einfacher Weise an verschieden große Ohren bzw. unterschiedliche Ohrformen angepasst werden. Der Elektrodenkopf bzw. die Elektroden liegen dabei stets mit einem definierten Druck auf der Haut auf.

In der Zeichnung ist ein Ausführungsbeispiel der Erfindung dargestellt. Es zeigen:
- Fig. 1: in perspektiver Ansicht eine Elektrodenanordnung gemäß der Erfindung,
- Fig. 2: die Ansicht einer Pinna (Ohrmuschel), in die eine Elektrodenanordnung gemäß Fig. 1 zur Aufbringung eines transkutanen Stimulationsreizes eingesetzt ist, wobei hier einige Teile der Elektrodenanordnung zwecks Übersichtlichkeit der Darstellung nicht gezeigt sind,
- Fig. 3: in perspektivischer Darstellung das Halteelement der Elektrodenanordnung gemäß Fig. 1,
- Fig. 4: in perspektivischer Darstellung einen Abschnitt der Haltestange der Elektrodenanordnung gemäß Fig. 1,
- Fig. 5: in perspektivischer Darstellung den Elektrodenträger samt Elektroden der Elektrodenanordnung gemäß Fig. 1,
- Fig. 6: in der Seitenansicht das Halteelement der Elektrodenanordnung gemäß Fig. 1,
- Fig. 7: in der Seitenansicht eine Elektrode der Elektrodenanordnung gemäß Fig. 1,
- Fig. 8: schematisch in geschnittener Darstellung einen Teil des Halteelements sowie einen Teil der Haltestange der Elektrodenanordnung, wobei die Haltestange noch nicht montiert ist, und
- Fig. 9: schematisch einen Stecker der Elektrodenanordnung, über den die Elektroden mit Strom versorgt werden.

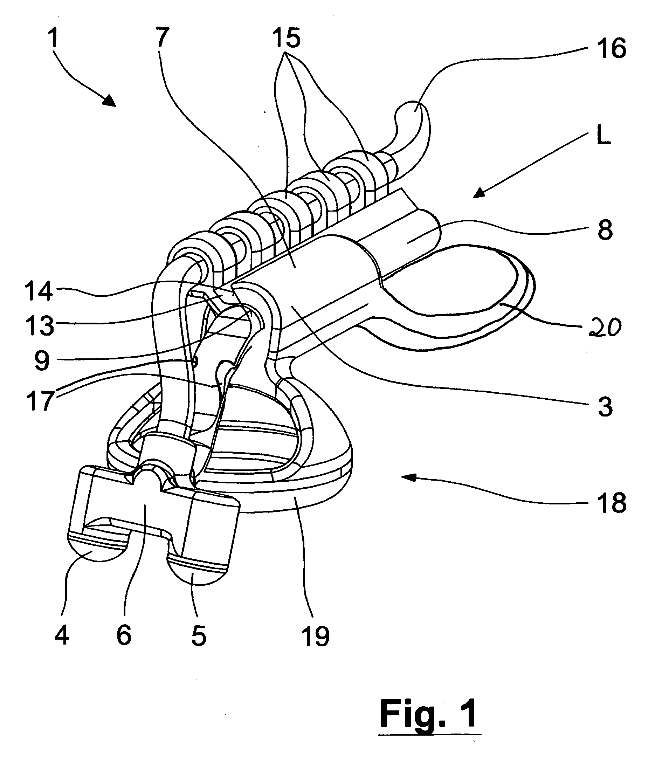

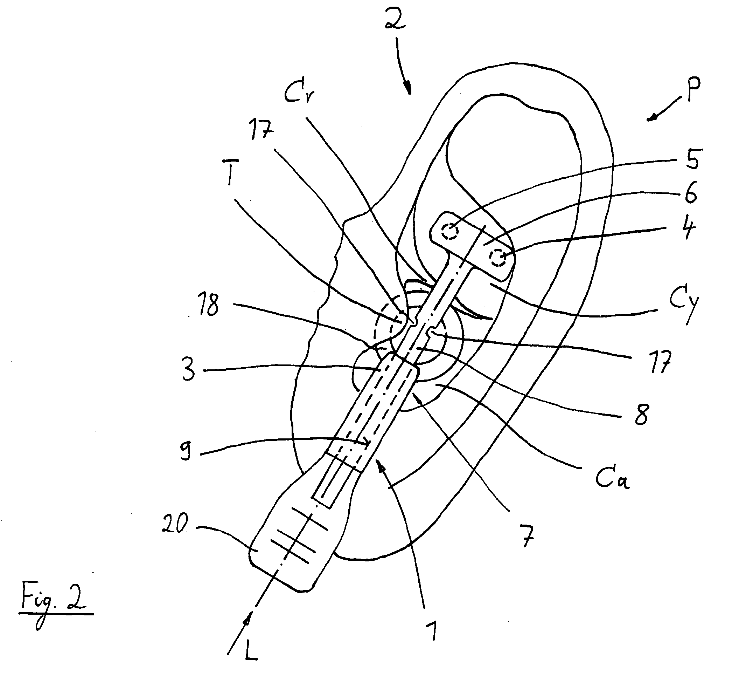

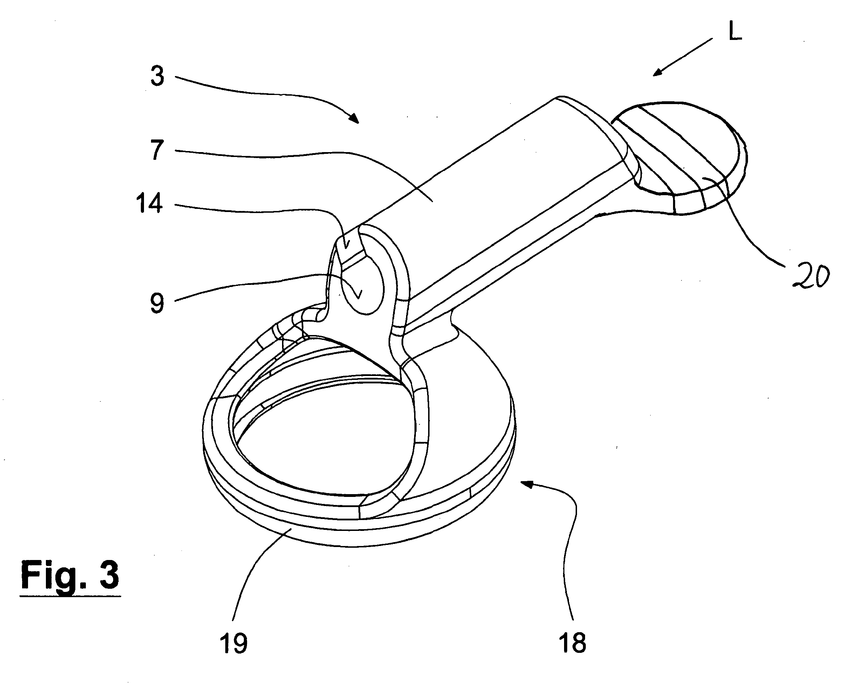

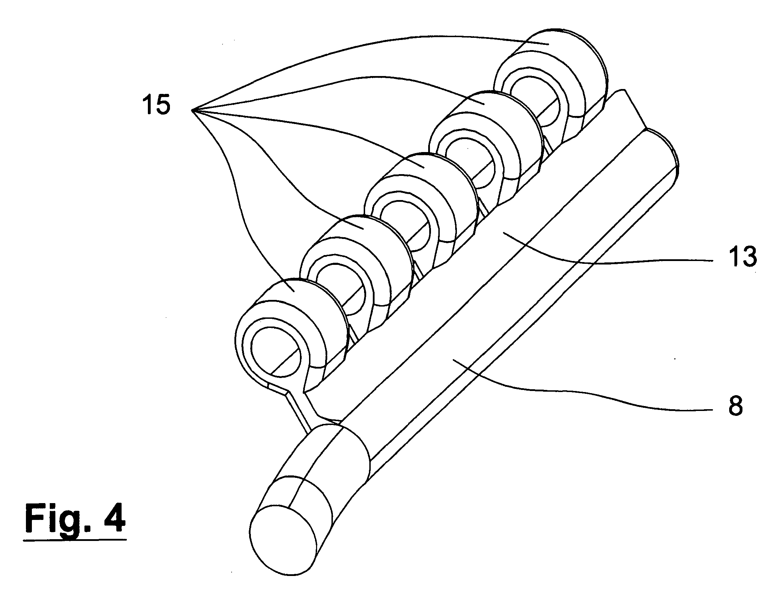

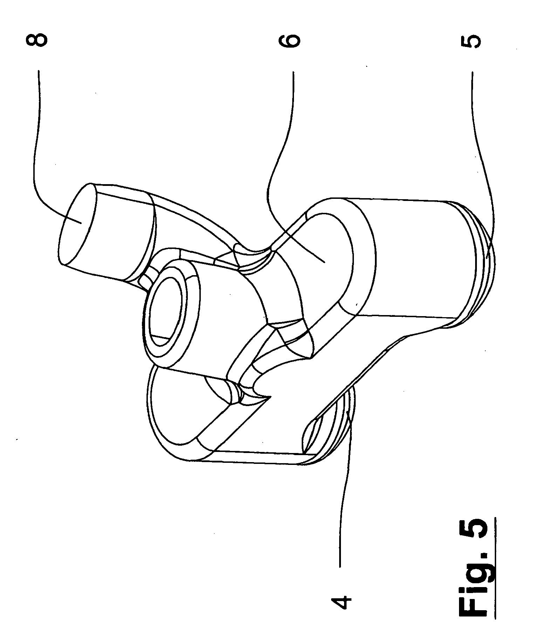

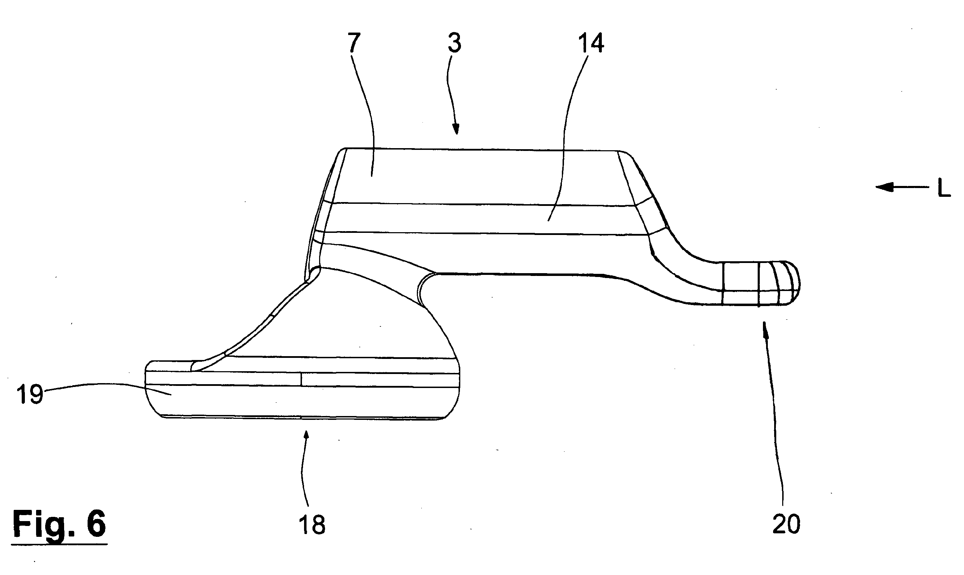

In Fig. 1 ist eine Elektrodenanordnung 1 in Form einer Otoplastik dargestellt, die in ein in Fig. 2 dargestelltes Ohr 2 eines Menschen eingesetzt werden kann, um eine transkutane Elektrostimulation der Hautoberfläche im Bereich des Ohres vornehmen zu können. Teile der Elektrodenanordnung 1 sind in den Figuren 3 bis 6 zu sehen.

Mit der Elektrodenanordnung 1 kann konkret auf einen Oberflächenbereich des Ohres eine transkutane elektrische Nervenstimulation vorgenommen werden. Hierfür weist die Elektrodenanordnung eine Stimulationselektrode und eine Referenzelektrode (s. unten) auf, zwischen denen ein elektrisches Potential erzeugt wird; die hierfür nötigen Mittel sind im Stand der Technik hinlänglich bekannt, so dass sie hier nicht weiter beschrieben werden müssen. Exemplarisch wird auf die DE 10 2005 003 735 B4 der Anmelderin verwiesen und hierauf ausdrücklich Bezug genommen.

Die Elektrodenanordnung 1 hat als wesentliche Baubestandteile ein Halteelement 3 sowie eine Haltestange 8. Die Haltestange 8 trägt an einem axialen Ende einen Elektrodenträger 6, der mit zwei Elektroden 4, 5 versehen ist, nämlich mit einer Stimulationselektrode 4 und einer (baugleichen) Referenzelektrode 5. Der Elektrodenträger 6 hält die beiden Elektroden 4, 5 auf einem exakten gewünschten Abstand und ist zwischen den Elektroden 4, 5 als Brückenelement ausgebildet. Das Halteelement 3 hat einen zentralen Abschnitt, der von einer Linearführung 7 dominiert wird. Hierbei handelt es sich um einen sich in eine Längsrichtung L erstreckenden Materialabschnitt mit stabartiger Form, in den eine kreisförmige Ausnehmung 9 eingeformt ist. Am vorderen Ende der Linearführung 7 ist ein Auflageteil 18 angeformt; am hinteren Ende ist eine Wangenauflage 20 angeformt. Der Auflageteil 18 weist einen ringförmigen Abschnitt 19 auf; die Wangenauflage 20 ist vorliegend als flacher Abschnitt ausgeführt und dient als zusätzliche Stabilisierung der Auflage der Elektrodenanordnung 1.

Wesentlich ist dabei, dass das Halteelement 3 mittels der Linearführung 7 in der Lage ist, die Haltestange 8 in Richtung der Längsachse L linear zu verschieben.

Damit wird es möglich, den Abstand zwischen dem Elektrodenkopf 6 und somit der Elektroden 4, 5 und insbesondere dem Auflageteil 18 zu verändern und auf ein gewünschtes Maß einzustellen.

Diese Einstellmöglichkeit wird genutzt, um die Elektrodenanordnung nach Platzierung im Ohr 2 so zu verstellen, dass sie bei gutem Tragekomfort elastischen Halt im Ohr 2 findet.

Hierzu wird auf Fig. 2 verwiesen. Hier ist zu sehen, dass die Elektrodenanordnung 1 ins Ohr 2 eingesetzt und durch lineare Verschiebung der Haltestange 8 relativ zum Halteelement 3 so eingestellt wurde, dass sich die Elektrodenanordnung 1 elastisch aufgrund der Topographie des Ohrs 2 festgespannt hat. Konkret wurde die Elektrodenanordnung 1 so in der Pinna P des Ohrs 2 angeordnet, dass der Elektrodenkopf 6 im Bereich der Cymba conchae Cy zu liegen kommt, während der Auflageteil 18 mit seinem ringförmigen Abschnitt 19 im Bereich des Cavum conchae Ca aufliegt. Demgemäß kommt der ringförmige Abschnitt 19 unterhalb des Tragus T und des Crus helicis Cr zu liegen.

Infolge einer (materialbedingten) Federelastizität der Haltestange 8 bzw. wegen eines bei Bedarf in die Haltestange 8 integrierten Federelements (z. B. Federdrahts) spannt sich somit die Elektrodenanordnung 1 nach entsprechender linearer Einstellung der Haltestange 8 relativ zum Halteelement 3 elastisch vor, so dass ein hinreichender Halt im Ohr gegeben ist. Wie in Fig. 2 zu sehen ist, erlaubt die ringförmige Ausgestaltung des Abschnitts 19 ein weitgehend ungehindertes Hörempfinden.

Nochmals auf Fig. 1 zurückkommend, ist zu sehen, dass die Haltestange 8 mit Mitteln 13, 14 zur Verhinderung einer Verdrehung um die Längsachse relativ zum Halteelement 3 ausgestattet sind. Diese Mittel bestehen einmal aus einem Seitenfortsatz 13, der an der Haltestange 8 angeformt ist und sich radial von der Haltestange 8 nach außen erstreckt. Ferner werden die Mittel durch eine schlitzförmige Ausnehmung 14 gebildet, die - sich in Längsrichtung L erstreckend - in das Halteelement 3 eingebracht ist. Demgemäß kann sich die Haltestange 8 samt Seitenfortsatz 13 in der Linearführung 7 zwar in Längsrichtung L bewegen, allerdings nicht um die Längsachse L drehen.

Am radial außenliegenden Ende des Seitenfortsatzes 13 sind mehrere Haltemittel 15 in Form von ringförmigen Strukturen angeformt, die dazu dienen, das Kabel 16 (bevorzugt mit 3-ardiger Kevlarlitze und von biokompatiblem Mantelmaterial umgeben), das die Elektroden 4, 5 mit Strom versorgt, zugentlastet zu halten. Die Haltemittel 15 dienen also als Kabelführungsösen, in denen das Kabel 16 geführt und zugentlastet wird. Das Kabel 16 kann dabei beim Spritzgießen der einstückig ausgeführten Teile 8, 13 und 15 mit umspritzt werden. Bei der Herstellung des Elektrodenträgers 6 durch Spritzgießen kann in analoger Weise das Kabel 16 mit umspritzt werden, um eine hermetische Versiegelung aller Nähte zu erreichen.

Die Haltestange 8 weist über einen Bereich, der ca. die Hälfte bis zwei Drittel der Gesamterstreckung in Längsrichtung L entspricht, einen linearen, geraden Verlauf auf. Dieser ist im Querschnitt im wesentlichen korrespondierend zur Ausnehmung 9 im Halteelement 3 geformt, vorliegend also kreisförmig. An diesen linearen, geraden Abschnitt schließt sich - als Verbindungsabschnitt zum Elektrodenträger 6 - ein S-förmig gebogener Abschnitt an, der die Funktion hat, den Elektrodenträger 6 von der Höhe, in der sich die Linearführung 7 befindet, zur zu stimulierenden Hautoberfläche hinunter zu führen. Wie bereits erläutert, muss dies so erfolgen, dass der Elektrodenträger 6 elastisch gegen die Hauptoberfläche drückt und bevorzugt im Übrigen auch eine Vorspannung in Richtung der Längsachse L erzeugt.

Hierfür wird auf ein Material für die Haltestange zurückgegriffen, die die gewünschte Elastizität aufweist. Möglich ist es auch, dass in den gebogenen bzw. gekröpften Abschnitt der Haltestange 8 (bzw. über deren gesamte Erstreckung) ein Federelement integriert wird. Hierbei kann es sich beispielsweise um einen Federdraht handeln, der beim Spritzgießen der Haltestange 8 mit umspritzt wird.

Dabei erfolgen die Werkstoffwahl, gegebenenfalls die Integration eines Federelements und die geometrische Ausformung der Haltestange fachmännisch derart, dass eine gewünschte Elastizität vorliegt.

Damit sich in Richtung normal auf die zu stimulierende Hautoberfläche eine hinreichende elastische Andruckkraft ergibt, der Elektrodenträger 6 indes in biegeweicher Anbindung um eine zur Längsrichtung L senkrechten Achse relativ zum Halteelement 3 gehalten wird - so dass sich der Elektrodenträger 6 in optimaler Weise der Oberflächentopographie der zu stimulierenden Hautoberfläche anpassen kann -, ist gemäß einer besonderen Ausgestaltung vorgesehen, dass im Bereich der S-förmigen Ausbildung der Haltestange 8 seitlich zwei Einkerbungen bzw. Einschnitte 17 in die Haltestange 8 eingebracht sind (s. hierzu Fig. 1 und Fig. 2). Damit wird die Haltestange 8 um eine Achse, die senkrecht auf der Längsachse steht (nämlich um die Achse, die in Fig. 2 senkrecht auf der Zeichenebene steht) biegeweicher, ohne dass die Elastizität der Haltestange 8 normal zur zu stimulierenden Hautoberfläche wesentlich beeinträchtigt wird.

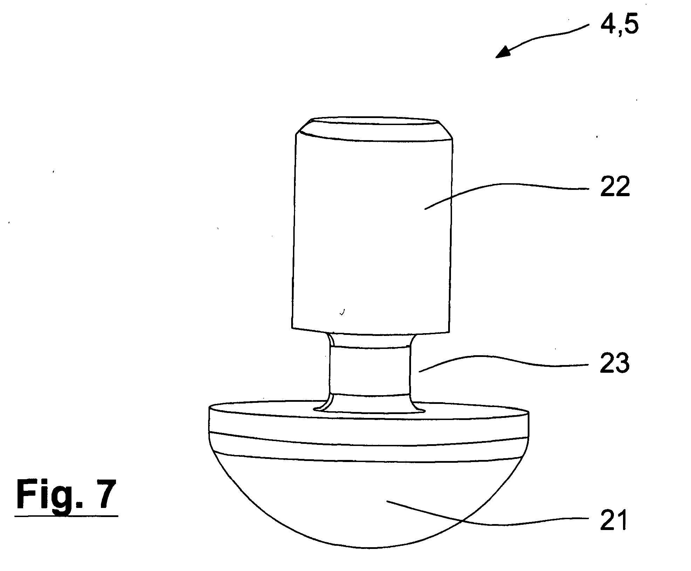

In Fig. 7 ist eine Elektrode 4, 5 in der Seitenansicht zu sehen, die im Elektrodenträger 6 durch Umspritzen fixiert ist. Die Elektrode weist einen Kontaktabschnitt 21 auf, der zur Kontaktnahme mit der zu stimulierenden Haut vorgesehen ist. Weiterhin hat die Elektrode 4, 5 einen stiftförmigen Verankerungsabschnitt 22, der im Material des Elektrodenträgers 6 fixiert wird. Zwischen Kontaktabschnitt 21 und Verankerungsabschnitt 22 ist ein Einschnitt 23 (Eindrehung) vorhanden, der einen axialen Hinterschnitt darstellt, der für eine formschlüssige axiale Fixierung der Elektrode 4, 5 im Elektrodenträger 6 sorgt. Es kann auch vorgesehen werden - was allerdings nicht dargestellt ist -, dass an einer Umfangsstelle des stiftförmigen Verankerungselements 22 eine Abflachung angeordnet ist, so dass nach dem Umspritzen der Elektrode 4, 5 bei der Herstellung des Elektrodenträgers 6 auch eine Drehung um die Längsachse der Elektrode 4, 5 verhindert wird. Die Elektroden 4, 5 bestehen bevorzugt aus einer Titan-Aluminium-Legierung (TiA16V4).

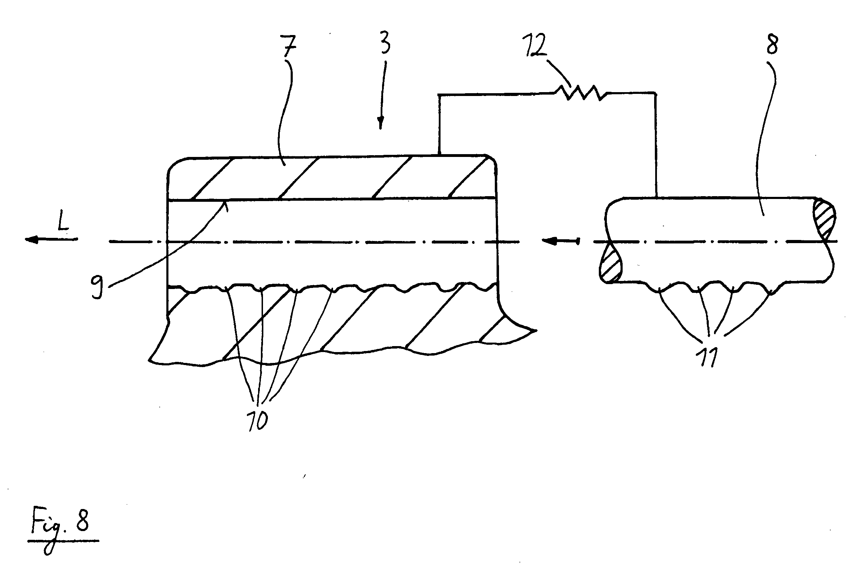

In Fig. 8 ist ein weiterer relevanter Aspekt der Elektrodenanordnung 1 schematisch dargestellt. Es ist zwar grundsätzlich möglich, die Abmessungen der Ausnehmung 9 im Halteelement 3 und des linearen Teils der Haltestange 8 so zu tolerieren, dass infolge einer Presspassung ein geeigneter Widerstand gegen die lineare Verschiebung der Haltestange 8 relativ zum Halteelement 3 gegeben ist, d. h. eine Linearverschieblichkeit ohne zu großen Kraftaufwand gegeben ist, allerdings auch eine hinreichende Haltekraft, so dass nach der Einstellung der Elektrodenanordnung diese im Ohr in Position gehalten wird.

Vorteilhaft ist indes eine Ausgestaltung, wie sie Fig. 8 zeigt. Hiernach sind Rastmittel 10, 11 in der Linearführung 7 vorgesehen, die es erlauben, die Haltestange 8 relativ zum Halteelement 3 in definierten Positionen einrastend festzulegen. Hierzu weist im Ausführungsbeispiel nach Fig. 8 die Ausnehmung 9 der Linearführung 7 zumindest an einer Umfangsstelle eine Anzahl von gleichmäßig beabstandeten nutförmigen Ausnehmungen 10 auf. Korrespondierend weist die Haltestange 8 eine Anzahl von Vorsprüngen 11 auf, die in gleichen Abständen angeordnet sind. Natürlich können auch umgekehrt die Ausnehmungen in der Haltestange 8 und die Vorsprünge in der Linearführung 7 angeordnet sein. Demgemäß können die Teile 3 und 8 in einer Anzahl von Rastpositionen relativ zueinander verrasten.

Wird nun die Haltestange 8 in die Ausnehmung 9 eingeschoben (s. Pfeil in Fig. 8), entstehen aufgrund der Materialelastizität beim Passieren der Vorsprünge 11 an der nutförmigen Ausnehmung 10 Rastpositionen, in denen die Linearführung 7 jeweilige Verharrungszustände findet, die nur durch Aufbringung einer hinreichenden Kraft in Richtung der Längsachse L überwunden werden können. Demgemäß kann die Haltestange 8 in einer gewünschten Rastposition in der Linearführung 7 platziert werden. Somit wird ein definierter schrittweiser Widerstand bei der translatorischen Verschiebung der Haltestange 8 relativ zum Halteelement 3 realisiert.

Nur schematisch dargestellt sind Federmittel 12, die zwischen der Haltestange 8 und dem Halteelement 3 wirksam sind und die eine federvorspannende Wirkung erzeugen können, um den Elektrodenträger 6 samt Elektroden 4, 5 elastisch vorgespannt zu halten. Die Federmittel 12 können beispielsweise als Gummiband, ausgestaltet sein, das in lineare Verschiebungsrichtung der Linearführung 7 wirksam ist.

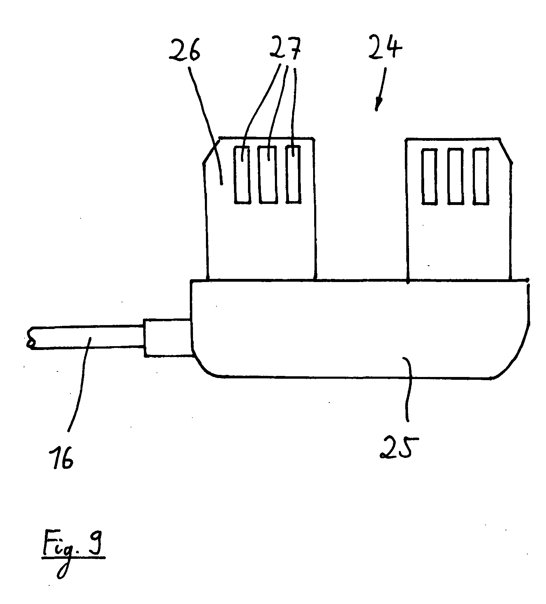

In Fig. 9 ist ein Stecker 24 zu sehen, mit dem der mechanische und elektrische Kontakt zu einem (nicht dargestellten) Stimulationsgerät hergestellt wird. In einem Grundkörper 25 wird eine Platine 26 fixiert, gleichermaßen das Kabel 16. Jeder Stecker der Platine 26 hat drei Kontaktstellen 27. Die Platine 26 wird in das Stimulatiönsgerät eingesteckt. Über die Kontaktstellen 27 und die entsprechenden Gegenstücke im Stimulationsgerät wird der elektrische Kontakt hergestellt. Durch eine nicht-symmetrische Ausgestaltung kann ein nicht korrektes Einstecken des Steckers 24 in das Stimulationsgerät verhindert werden.

### Bezugszeichenliste:

- 1: Elektrodenanordnung
- 2: Ohr
- 3: Halteelement
- 4: Elektrode (Stimulationselektrode)
- 5: Elektrode (Referenzelektrode)
- 6: Elektrodenträger
- 7: Linearführung
- 8: Haltestange
- 9: Ausnehmung
- 10, 11: Rastmittel
- 10: nutförmige Ausnehmung
- 11: Vorsprung
- 12: Federmittel
- 13, 14: Mittel zur Verhinderung einer Verdrehung
- 13: Seitenfortsatz
- 14: Ausnehmung
- 15: Haltemittel
- 16: Kabel
- 17: Einkerbung / Einschnitt
- 18: Auflageteil
- 19: ringförmiger Abschnitt
- 20: Wangenauflage
- 21: Kontaktabschnitt
- 22: stiftförmiger Verankerungsabschnitt
- 23: Einschnitt
- 24: Stecker
- 25: Grundkörper
- 26: Platine
- 27: Kontaktstelle

- L: Längsachse

- Ca: Cavum conchae
- Cy: Cymba conchae
- T: Tragus
- Cr: Crus helicis
- P: Pinna

## Patentansprüche

1. Elektrodenanordnung (1) zur Aufbringung eines transkutanen elektrischen Stimulationsreizes auf die Oberfläche eines Abschnittes des menschlichen Ohrs (2), die ein am oder im Ohr (2) anbringbares Halteelement (3) sowie mindestens eine Elektrode (4, 5) aufweist, die in einem Elektrodenträger (6) angeordnet ist,
wobei
das Halteelement (3) ein Auflageteil (18) umfasst, das zur Auflage im Cavum conchae (Ca) des Ohrs (2) ausgebildet und vorgesehen ist, und das Halteelement (3) eine Linearführung (7) aufweist, in der eine Haltestange (8) in Richtung einer Längsachse (L) des Halteelements (3) linear verschieblich angeordnet ist, wobei an der Haltestange (8) der Elektrodenträger (6) angeordnet ist.

2. Elektrodenanordnung nach Anspruch 1, **dadurch gekennzeichnet, dass** der Elektrodenträger (6) an einem axialen Ende der Haltestange (8) angeordnet ist.

3. Elektrodenanordnung nach Anspruch 1 oder 2, **dadurch gekennzeichnet, dass** der Elektrodenträger (6), insbesondere als Elektrodenkopf ausgebildet, mindestens eine Stimulationselektrode (4) und mindestens eine Referenzelektrode (5) aufweist.

4. Elektrodenanordnung nach einem der Ansprüche 1 bis 3, **dadurch gekennzeichnet, dass** die Linearführung (7) des Halteelements (3) durch eine Ausnehmung (9) gebildet wird, die in einem Schnitt senkrecht zur Längsachse (L) entlang der Längsachse (L) eine gleichbleibende Form aufweist, insbesondere eine Kreisform, wobei die Haltestange (8) zumindest über einen Teil ihrer Erstreckung vorzugsweise einen kreisförmigen Querschnitt aufweist.

5. Elektrodenanordnung nach einem der Ansprüche 1 bis 4, **dadurch gekennzeichnet, dass** Rastmittel (10, 11) zwischen der Linearführung (7) und der Haltestange (8) wirksam angeordnet sind, so dass die Haltestange (8) relativ zur Linearführung (7) entlang der Längsachse (L) in vorgegebenen Relativpositionen verrastbar angeordnet werden kann.

6. Elektrodenariordnung nach Anspruch 5, **dadurch gekennzeichnet, dass** die Rastmittel (10, 11) mindestens eine nutförmige Ausnehmung (10) in der Linearführung (7) umfasst sowie mindestens einen sich radial nach außen erstreckenden Vorsprung (11) in der Haltestange (8), wobei die Vorsprünge (11) vorzugsweise als Wellenbahn mit einer Anzahl radialer Erhebungen ausgebildet sind.

7. Elektrodenanordnung nach einem der Ansprüche 1 bis 6, **dadurch gekennzeichnet, dass** Federmittel (12) zwischen der Linearführung (7) und der Haltestange (8) wirksam angeordnet sind, so dass die Haltestange (8) relativ zur Linearführung (7) in Richtung der Längsachse (L) elastisch vorspannbar ist.

8. Elektrodenanordnung nach einem der Ansprüche 1 bis 7, **dadurch gekennzeichnet, dass** Mittel (13, 14) zur Verhinderung einer Verdrehung der Haltestange (8) relativ zur Linearführung (7) um die Längsachse (L) vorhanden sind.

9. Elektrodenanordnung nach Anspruch 8, **dadurch gekennzeichnet, dass** die Mittel (13, 14) durch einen sich radial von der Haltestange (8) weg erstreckenden Seitenfortsatz (13) gebildet werden, wobei in der Linearführung (7) eine korrespondierende Ausnehmung (14) für den Seitenfortsatz (13) ausgebildet ist.

10. Elektrodenanordnung nach Anspruch 9, **dadurch gekennzeichnet, dass** der Seitenfortsatz (13) sowie die Ausnehmung (14) in einem Schnitt senkrecht zur Längsachse (L) eine rechteckige Form aufweisen.

11. Elektrodenanordnung nach einem der Ansprüche 1 bis 10, **dadurch gekennzeichnet, dass** die Haltestange (8) in ihrem axialen Endbereich, in dem der Elektrodenträger (6) angeordnet ist, eine S-förmige Ausbildung hat.

12. Elektrodenanordnung nach einem der Ansprüche 1 bis 11, **dadurch gekennzeichnet, dass** die Haltestange (8) in ihrem axialen Endbereich, in dem der Elektrodenträger (6) angeordnet ist, mit einem Federelement, insbesondere mit einem Federdraht oder einer Blattfeder, verstärkt ist.

13. Elektrodenanordnung nach einem der Ansprüche 1 bis 12, **dadurch gekennzeichnet, dass** die Haltestange (8) in ihrem axialen Endbereich, in dem der Elektrodenträger (6) angeordnet ist, mindestens eine seitliche Einkerbung oder mindestens einen seitlichen Einschnitt (17) aufweist, um im axialen Endbereich die Biegesteifigkeit der Haltestange (8) um eine zur Längsachse (L) senkrechte Richtung zu reduzieren.

14. Elektrodenanordnung nach einen der Ansprüche 1 bis 13, **dadurch gekennzeichnet, dass** das Auflageteil (18) einen ringförmigen Abschnitt (19) hat, insbesondere einen kreisringförmigen Abschnitt oder einen ovalen Ringabschnitt.

15. Elektrodenanordnung nach einem der Ansprüche 1 bis 14, **dadurch gekennzeichnet, dass** das Halteelement (3) eine Wangenauflage (20) umfasst, die an dem vom Elektrodenträger (6) entfernten Ende der Elektrodenanordnung (1) am Halteelement (3) angeordnet ist.

## Claims

1. Electrode arrangement (1) for the application of a transcutaneous electric stimulation stimulus onto the surface of a section of the human ear (2), which comprises a holding element (3) to be arranged at or in the ear (2) as well as at least one electrode (4, 5), which electrode (4, 5) is arranged in an electrode carrier (6), wherein the holding element (3) comprises a resting part (18), which resting part is designed and provided for the application onto the Cavum conchae (Ca) of the ear (2) and the holding element (3) comprises a linear guide (7) in which a holding bar (8) is arranged linear movably in the direction of a longitudinal axis (L) of the holding element (3), wherein the electrode carrier (6) is arranged at the holding bar (8).

2. Electrode arrangement according to claim 1, **characterized in that** the electrode carrier (6) is arranged at an axial end of the holding bar (8).

3. Electrode arrangement according to claim 1 or 2, **characterized in that** the electrode carrier (6), designed especially as electrode head, comprises at least one stimulation electrode (4) and at least one reference electrode (5).

4. Electrode arrangement according to one of claims 1 to 3, **characterized in that** the linear guide (7) of the holding element (3) is formed by a recess (9) which recess (3) has a constant shape, especially a circular shape, along the longitudinal axis (L) in a section perpendicular to the longitudinal axis (L), wherein the holding bar (8) comprises preferably a circular shaped cross section at least along a part of its extension.

5. Electrode arrangement according to one of claims 1 to 4, **characterized in that** latching means (10, 11) are effectively arranged between the linear guide (7) and the holding bar (8), so that the holding bar (8) can be arranged lockable relatively to the linear guide (7) along the longitudinal axis (L) in predetermined relative positions.

6. Electrode arrangement according to claim 5, **characterized in that** the latching means (10, 11) comprise at least one groove-shaped recess (10) within the linear guide (7) as well as at least one protrusion (11) within the holding bar (8), which protrusion extends radial outward, wherein the protrusions (11) are designed preferably as a wave path with a plurality of radial elevations.

7. Electrode arrangement according to one of claims 1 to 6, **characterized in that** spring means (12) are effectively arranged between the linear guide (7) and the holding bar (8), so that the holding bar (8) can be biased elastically relative to the linear guide (7) in the direction of the longitudinal axis (L).

8. Electrode arrangement according to one of claims 1 to 7, **characterized in that** means (13, 14) are arranged for preventing of rotation of the holding bar (8) relatively to the linear guide (7) around the longitudinal axis (L).

9. Electrode arrangement according to claim 8, **characterized in that** the means (13, 14) are formed by a lateral extension (13), which lateral extension is radial extending from the holding bar (8), wherein in the linear guide (7) a corresponding recess (14) for the lateral extension (13) is arranged.

10. Electrode arrangement according to claim 9, **characterized in that** the lateral extension (13) as well as the recess (14) have a square form in a section perpendicular to the longitudinal axis (L).

11. Electrode arrangement according to one of claims 1 to 10, **characterized in that** the holding bar (8) has a S-form design in its axial end region, in which end region the electrode carrier (6) is arranged.

12. Electrode arrangement according to one of claims 1 to 11, **characterized in that** the holding bar (8) is reinforced in its axial end region with a spring element, particularly with a spring wire or a leaf spring, in which end region the electrode carrier (6) is arranged.

13. Electrode arrangement according to one of claims 1 to 12, **characterized in that** the holding bar (8) comprises at least one collateral notch or at least one collateral insection (17) in its axial end region, in which end region the electrode carrier (6) is arranged, to reduce the bending stiffness of the holding bar (8) in the axial end region around a direction, which is perpendicular to the longitudinal axis (L).

14. Electrode arrangement according to one of claims 1 to 13, **characterized in that** the resting part (18) has a ring-shaped section (19), particularly a circular ring-shaped section or an oval ring section.

15. Electrode arrangement according to one of claims 1 to 14, **characterized in that** the holding element (3) comprises a cheek rest (20), which cheek rest is arranged at the holding element (3) at the end of the electrode arrangement (1) remote from the electrode carrier (6).

## Revendications

1. Ensemble (1) d'électrodes destiné à appliquer une excitation électrique transcutanée de stimulation sur la surface d'une partie de l'oreille humaine (2) et présentant un élément de maintien (3) apte à être placé sur ou dans l'oreille (2) ainsi qu'au moins une électrode (4, 5) disposée dans un porte-électrodes (6),
l'élément de maintien (3) comportant une partie de pose (18) configurée et prévue pour être placée dans le Cavum conchae (Ca) de l'oreille (2),
l'élément de maintien (3) présentant un guide linéaire (7) dans lequel une tige de maintien (8) est disposée à coulissement linéaire dans la direction de l'axe longitudinal (L) de l'élément de maintien (3),
le porte-électrodes (6) étant disposé sur la tige de maintien (8).

2. Ensemble d'électrodes selon la revendication 1, **caractérisé en ce que** le porte-électrodes (6) est disposé à une extrémité axiale de la tige de maintien (8).

3. Ensemble d'électrodes selon les revendications 1 ou 2, **caractérisé en ce que** le porte-électrodes (6) configuré en particulier comme tête d'électrode présente au moins une électrode de stimulation (4) et au moins une électrode de référence (5).

4. Ensemble d'électrodes selon l'une des revendications 1 à 3, **caractérisé en ce que** le guide linéaire (7) de l'élément de maintien (3) est formé par une découpe (9) qui présente dans une coupe perpendiculaire à l'axe longitudinal (L) une forme constante le long de l'axe longitudinal (L), en particulier la forme d'un cercle, la tige de maintien (8) présentant de préférence une section transversale circulaire sur au moins une partie de son extension.

5. Ensemble d'électrodes selon l'une des revendications 1 à 4, **caractérisé en ce que** des moyens d'encliquetage (10, 11) sont disposés de manière à agir entre le guide linéaire (7) et la tige de maintien (8) de telle sorte que la tige de maintien (8) puisse être disposée de manière encliquetable en des positions relatives prédéterminées par rapport au guide linéaire (7) le long de l'axe longitudinal (L).

6. Ensemble d'électrodes selon la revendication 5, **caractérisé en ce que** les moyens d'encliquetage (10, 11) comprennent au moins une découpe (10) en forme de rainure ménagée dans le guide linéaire (7) ainsi qu'au moins une saillie (11) qui s'étend radialement vers l'extérieur dans la tige de maintien (8), les saillies (11) étant de préférence configurées comme pistes ondulées présentant plusieurs rehaussements radiaux.

7. Ensemble d'électrodes selon l'une des revendications 1 à 6, **caractérisé en ce que** des moyens élastiques (12) sont disposés de manière à agir entre le guide linéaire (7) et la tige de maintien (8) de telle sorte que la tige de maintien (8) puisse être précontrainte élastiquement par rapport au guide linéaire (7) dans la direction de l'axe longitudinal (L) .

8. Ensemble d'électrodes selon l'une des revendications 1 à 7, **caractérisé en ce que** des moyens (13, 14) qui empêchent la tige de maintien (8) de tourner par rapport au guide linéaire (7) autour de l'axe longitudinal (L) sont prévus.

9. Ensemble d'électrodes selon la revendication 8, **caractérisé en ce que** les moyens (13, 14) sont formés par un appendice latéral (13) qui s'éloigne radialement de la tige de maintien (8), une découpe (14) correspondante étant formée dans le guide linéaire (7) pour l'appendice latéral (13).

10. Ensemble d'électrodes selon la revendication 9, **caractérisé en ce que** l'appendice latéral (13) ainsi que la découpe (14) présentent en coupe perpendiculaire à l'axe longitudinal (L) la forme d'un rectangle.

11. Ensemble d'électrodes selon l'une des revendications 1 à 10, **caractérisé en ce que** la tige de maintien (8) est configurée en S dans sa partie d'extrémité axiale dans laquelle le porte-électrodes (6) est disposé.

12. Ensemble d'électrodes selon l'une des revendications 1 à 11, **caractérisé en ce que** la tige de maintien (8) est renforcée par un élément élastique, en particulier un fil de ressort ou une lame de ressort, dans sa partie d'extrémité axiale dans laquelle le porte-électrodes (6) est disposé.

13. Ensemble d'électrodes selon l'une des revendications 1 à 12, **caractérisé en ce que** la tige de maintien (8) présente au moins une entaille latérale ou au moins une découpe latérale (17) dans sa partie d'extrémité axiale dans laquelle le porte-électrodes (6) est disposé, pour réduire la rigidité en flexion de la tige de maintien (8) autour d'une direction perpendiculaire à l'axe longitudinal (L) dans la partie d'extrémité axiale.

14. Ensemble d'électrodes selon l'une des revendications 1 à 13, **caractérisé en ce que** la partie de pose (18) présente une partie annulaire (19), en particulier une partie en forme d'anneau circulaire ou une partie annulaire ovale.

15. Ensemble d'électrodes selon l'une des revendications 1 à 14, **caractérisé en ce que** l'élément de maintien (3) présente un appui en joue (20) disposé sur l'élément de maintien (3) à l'extrémité de l'ensemble (1) d'électrodes distante du porte-électrodes (6).
